# EUROPEAN PATENT APPLICATION

(11) **EP 1 882 943 A1**
(43) Date of publication of application: **30.01.2008**
(21) Application number: 06015675.9
(22) Date of filing: 27.07.2006
(51) Int. Cl.: G01N 33/558, G01N 33/569, G01N 33/53, B01L 3/00, A61B 10/00

(54) **Saliva immunochromatographic detection cup**

(71) Applicant: The Jordanian Pharmaceutical Manufacturing Co., 11710 Naor (JO)
(72) Inventor: Badwan, Adnan, Dr., Amman 11185 (JO); Mohammed, Murshed Abedel-qader, Amman 11118 (JO)
(74) Representative: Winkler, Andreas Fritz Ernst

(57) **Abstract**

The present invention refers to an immunochromatographic detection cup and its use for detecting human HIV-1, HIV-2, HCV and *H. pylori* antibodies in oral fluid.

## Description

The present invention refers to an immunochromatographic detection cup and its use for detecting human HIV-1, HIV-2, HCV and *H. pylori* antibodies in oral fluid.

### Background of the Invention

Testing for HIV is an essential component in the diagnosis and treatment of persons infected with the virus, in screening of blood for transfusion, in surveillance and in HIV/AIDS related research. Thus accurate and cost-effective testing is of great importance in combating the spread of HIV. It is imperative that tests for the diagnosis of HIV infection be as accurate as possible, given the serious ethical, legal and social issues that accompany HIV infection.

The use of body fluids other than blood as specimens for detecting antibodies to HIV has been reported to have potential as an alternative medium for HIV testing. The use of urine samples and also samples derived from the oral cavity (saliva) may be attractive due to the ease of sample collection, possible cost savings, better safety (against needle stick injuries) and higher compliance rates. Assays for these types of specimens can be a useful alternative when it is difficult or impossible to test for HIV in blood samples. It may be that blood cannot be drawn for religious reasons or difficulties may be experienced in collecting blood samples in hard to reach places where it is, nevertheless, important to have epidemiological surveillance. Moreover, urinary and salivary samples are the more safety type of specimens in case of HIV infection.

Today new HIV tests have been developed for use with urine specimens or saliva. Urine contains very low levels of IgG, frequently around 1 mg/L, and therefore very sensitive techniques are required to detect specific antibody. Thus, to date, the tests for urine specimens have been based on ELISA and Western blot techniques, however, efforts are being made to develop simple and/or rapid tests for the detection of antibody to HIV in urine specimens.

Oral fluid specimens consist mostly of saliva, which predominantly contains IgA class antibody, and oral mucosal transudates, which mostly contain IgG, and therefore also have much lower levels of IgG than serum. The levels of IgG normally found in oral fluid specimens (approximately 15 mg/l) are, however, higher than in urine specimens and innovative simple/rapid technology that has been shown to be effective for whole blood, serum and plasma, e.g. lateral flow through a chromatographic membrane, has been developed for use with these specimens¹.

In recent years the *in vitro* diagnostics (IVD) industry has made enormous efforts to develop immunochromatographic tests. Such tests have found applications in both clinical and non-clinical fields². A clinical utility of this test format has been shown for more than 150 different analytes, and many of them are target now of commercially available diagnostic products ³. The wide range of applications for such devices has been reviewed^{2,4}.

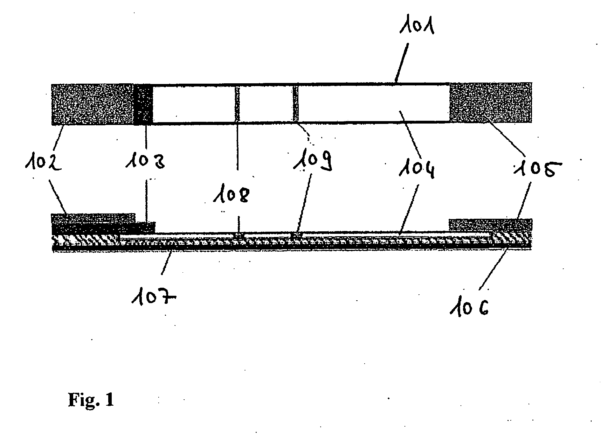

Rapid immunochromatographic test devices, e.g. in the form of a test strip, are made up of a number of components (see Fig. 1). Such a test strip 101 commonly includes a sample pad 102, a conjugate pad 103, a membrane 104, e.g. a nitrocellulose membrane, and an absorbent pad 105. The membrane 104 is usually attached by means of an adhesive 106 to a supporting backing 107, e.g. made of plastic. In practice, the user dispense a patient sample (usually urine or whole blood) onto the sample pad 102.The sample then flows through the sample pad 102 into the conjugate pad 103, where it mixes with and releases the detector reagent. This mixture then flows across the membrane 104, where it binds with the test and control reagents located in the capture test zone 108 and negative control zone 109, respectively. When the mixture binds to the reagent that forms the test line, a positive result is indicated. The colour intensity of the test line is proportional to the concentration of analyte in the sample. Excess sample that flows beyond the test and control zones 108, 109 is taken up in the absorbent pad 105.

Rapid immunochromatographic test devices for diagnostic purposes are easy to operate and thus do not only contribute to the comfort of professional users, e.g. medical stuff, but also allow the operation by non-professionals users, e.g. most patients.

The object of the present invention is to provide a rapid immunochromatographic test device for detecting antibodies such as HIV in oral fluid.

### Summary of the Invention

The object of the present invention is solved by an oral fluid immunochromatographic detection cup comprising:
(a) a sample-collecting container;
(b) at least one test device inside the container, which test device comprises at least one capture antigen recognized by an antibody to be detected and/or at least one control antibody;
(c) at least one conjugate releasing pad separated from the test device, which conjugate releasing pad comprises at least one anti-antibody colloidal gold conjugate;
(d) at least two sample absorbent pads linked to the test device at different positions.

In one embodiment, the detection cup comprises.
(a) a sample-collecting container comprising at least two compartments;
(b) at least one test device inside each compartment, which test device comprises at least one capture antigen recognized by an antibody to be detected and/or at least one control antibody;
(c) at least one conjugate releasing pad inside each compartment separated from the test device, which conjugate releasing pad comprises at least one anti-antibody colloidal gold conjugate;
(d) at least two sample absorbent pads linked to each test device at different positions.

In one embodiment, the test device comprises a nitrocellulose membrane on which the capture antigen and/or the control antibody are immobilized.

In one embodiment, the antibody to be detected is a human antibody selected from HIV- 1 IgG, HIV-1 IgA, HIV-2 IgG, HIV-2 IgA, HCV IgG, HCV IgA, *H. pylori* IgG, and *H. pylori* IgA.

In one embodiment, the control antibody is anti-mouse IgG.

In one embodiment, the anti-antibody colloidal gold conjugate is mouse anti-human IgG or mouse anti-human IgA or specific antigen colloidal gold conjugate.

In one embodiment, the conjugate releasing pad is located at the internal surface of the container wall.

In one embodiment, a first sample absorbent pad is located at the bottom of the container and a second sample absorbent pad is located at the top of the container.

In a preferred embodiment, the absorbent sample pad at the top is fitted into a container cap.

In a particularly preferred embodiment, the absorbent sample pad at the top has the capacity of absorbing at least 15 ml of sample.

The object of the present invention is further solved by a use of the oral fluid immunochromatographic detection cup for detecting a human antibody selected from HIV-1 IgG, HIV-1 IgA, HIV-2 IgG, HIV-2 IgA, HCV IgG, HCV IgA, *H pylori* IgG, and *H pylori* IgA in oral fluid of a subject, preferably human.

In one embodiment, the detection cup is used for simultaneously detecting HIV-1 IgG, HIV-1 IgA, HIV-2 IgG, HIV-2 IgA, HCV IgG, and HCV IgA.

In another embodiment, the detection cup is used simultaneously detecting of HIV-1 IgG, HIV-1 IgA, HIV-2 IgG, HIV-2 IgA, HCV IgG, *H. pylori* IgG, and *H. pylori* IgA

In one embodiment, the detection cup is used for the diagnosis and monitoring of a subject infected with HIV-1, HIV-2, HCV and/or *H. pylori.*

Thus, by using a large volume of oral fluid as a sample (about 15 ml) instead of 17 µl of serum (concentration of IgG in saliva is about 1:400), the present invention provides a rapid immunochromatographic test device for detecting HIV-1, HIV-2, HCV and/or *H. pylori* antibodies in a patient's sample readily obtainable by non-invasive procedures.

### Detailed Description of the Invention

### Brief Description of the Figures

- Figure 1: shows top and side views of a typical rapid-flow immunochromatographic test device in the form of a test strip 101 including a sample pad 102, a conjugate pad 103, a membrane 104, an absorbent pad 105, an adhesive 106, a supporting backing 107, a test zone 108, and a control zone 109.
- Figure 2a: shows an assembly comprising a test strip 201, an absorbent sample pad 202, and an absorbent pad 205.
- Figure 2b: shows an immunochromatographic detection cup comprising a sample-collecting container 210, a cap 211 and the assembly shown in Fig. 2a.
- Figure 3: shows an immunochromatographic detection cup comprising a sample-collecting container 310 divided into two compartments by a divider 312 and a cap 311, wherein a first compartment comprises a test strip 301 and a second compartment comprises a test strip 301'.

### EXAMPLES

### EXAMPLE 1: Saliva Immunochromatographic Detection Cup

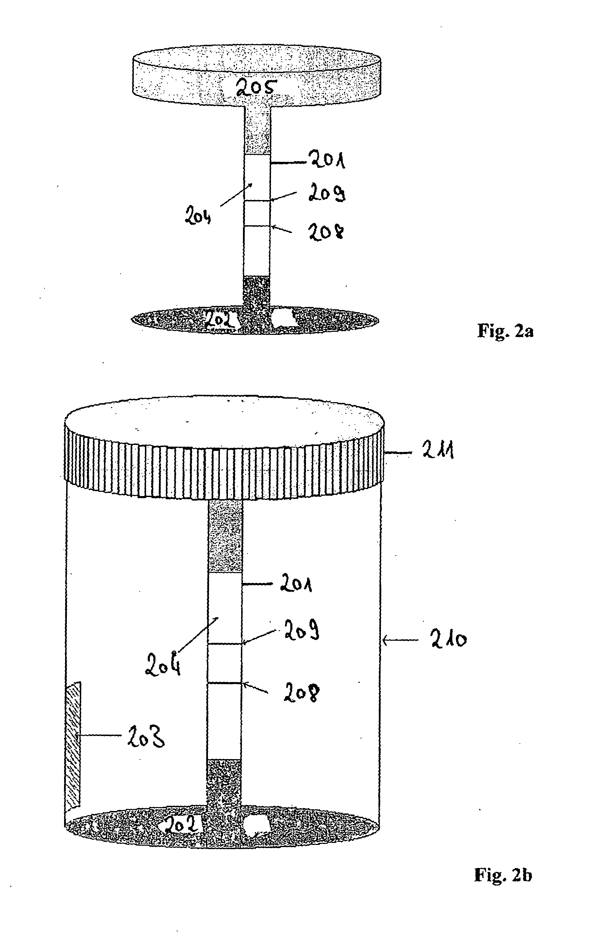

One embodiment of the rapid immunochromatographic detection system of the present invention is shown in Fig. 2a, b. The immunochromatographic detection cup comprises a sample-collecting container 210, actually the "cup", a cap 211 for closing the container, and a detection test strip 201 inserted onto the container 210, e.g. inside the transparent container wall. The test result can be read on the outer surface. The test strip 201 comprises a nitrocellulose membrane 204 and is linked via an absorbent pad 202 on the test strip 201 to an absorbent sample pad 202 placed on the bottom of the container 210. The absorbent sample pad 202 is designed to cover the inner surface of the container bottom. At the opposite end, the test strip 201 is linked via an absorbent pad 205 on the test strip to an absorbent pad 205 which is fitted into the cap 211 of the container and is designed to be thick enough to absorb more than 15 ml of sample. An anti-human immunoglobulin gold conjugate releasing pad 203 is fixed to the internal surface of the container wall. The anti-human immunoglobulin gold conjugate will begin releasing during addition of the sample into the container 210. Non-specific antibody is immobilized as a control zone 209 onto the nitrocellulose membrane 204 for non-specific capturing of the anti-human immunoglobulin gold conjugate, while specific antigens are immobilized as a sample zone 208 for specific capturing of the antibody to be detected. The antigens may be synthetic and/or recombinant. In the embodiment shown in Fig. 2a, b the sample zone 208 and the control zone 209 are realized by a sample and control line, respectively. More than one sample zone 208 and/or more than one control zone 209 are also contemplated.

### EXAMPLE 2: Compartment Saliva Immunochromatographic Detection Cup

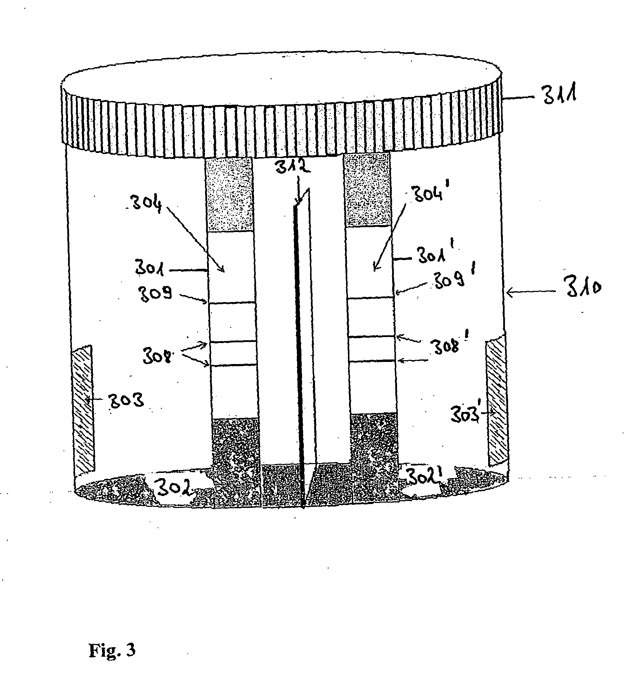

Another embodiment of the rapid immunochromatographic detection system of the present invention is shown in Fig. 3. In this embodiment, the immunochromatographic detection cup comprising a sample-collecting container 310 and a cap 311 for closing the container is divided into two compartments I and II by a divider 312. Each compartment comprises a detection test strip 301, 301' comprising a nitrocellulose membrane 304, 304' inserted into the container 310. Each of the test strips 301, 301' is linked with an absorbent pad 302, 302' placed on the bottom of the container 310. Each of the absorbent sample pads 302, 302' is designed to cover the inner surface of the compartment container bottom. At their opposite ends, the test strips 301, 301' are each linked to an absorbent pad 305 on the test strips 301, 301' which is fitted into the cap 311 of the container 310 and is designed to be thick enough to absorb more than 15 ml of sample. In each of the compartments, an anti-human immunoglobulin gold conjugate releasing pad 303, 303' is fixed to the internal surface of the container wall. The anti-human immunoglobulin gold conjugate will begin releasing during addition of the sample into the container 310. Non-specific antibody is immobilized as a control zone 309, 309' on each nitrocellulose membrane 304, 304' for non-specific capturing of the anti-human immunoglobulin gold conjugate, while specific antigens are immobilized as a sample zone 308, 308' onto each cellulose membrane 304, 304' for specific capturing of the antibody to be detected. The antigens may be synthetic and/or recombinant. In the embodiment shown in Fig. 3 the sample zone 308, 308' and the control zone 309, 309' are realized by a sample and control line, respectively. More than one sample zone 308, 308' and/or more than one control zone 209, 209' on each test strip 301, 301' are also contemplated. Furthermore, more than two compartments I and II as well as more than one test strip in each compartment are also contemplated.

### EXMAPLE 3: Oral HIV Antibody Detection Cup

The two compartment configuration (see Fig. 3 and Example 2 above) is used. The capacity of each of the compartments I and II is at least 7.5 ml of sample. Gold conjugate A is mouse anti-human IgG colloidal gold conjugate immobilized on the conjugate releasing pad 303 of compartment I of the sample collection container 310, and gold conjugate B is mouse anti-human IgA colloidal gold conjugate immobilized on the conjugate releasing pad 303' of compartment II. Three zones are located at each of the detection strips 301, 301'; a first sample zone 308, 308', a second sample zone 308, 308' and a control zone 309, 309'. The first sample zone 308, 308' is comprised of a mixture of recombinant and synthetic HIV-2 specific antigens while the second sample zone 308, 308' is comprised of a mixture of recombinant and synthetic HIV-1 specific antigens. The control zone 309, 309' of the two test strips 301, 301' is anti-mouse IgG.

In compartment I (IgG), the first sample zone 308 and the control region 309 turn into purple colour in case that HIV-2 IgG is present in the sample. The second sample zone 308 and the control zone 309 turn into purple colour in case that HIV-1 IgG is present in the sample. Only the control zone 309 turns into purple colour in case of HIV IgG free sample.

In compartment II (IgA), the first sample zone 308' and the control zone 309' turn into purple colour in case that HIV-2 IgA is present in the sample. The second sample zone 308' and the control zone 309' turn into purple colour in case that HIV-1 IgA is present in the sample. Only the control zone 309' turns into purple colour in case of HIV IgA free sample.

### EXAMPLE 4: Oral HCV Antibody Detection Cup

The two compartment configuration (see Fig. 3 and Example 2 above) is used. The capacity of each of the compartments I and II is at least 7.5 ml of sample. Gold conjugate A is mouse anti-human IgG colloidal gold conjugate immobilized on the conjugate releasing pad 303 of compartment I of the sample collection container 310, and gold conjugate B is mouse anti-human IgA colloidal gold conjugate immobilized onto the conjugate releasing pad 303' of compartment II of the sample collection container 310. Two zones are located at each the detection strips 301, 301' of the two compartments; a sample zone 308, 308' and a control zone 309, 309'. The sample zone 308, 308' is comprised of a mixture of recombinant and synthetic HCV specific antigens. The control zone of the two test strips 301, 301'is anti-mouse IgG.

In compartment I (IgG), the sample zone 308 and the control zone 309 turn into purple colour in case that HCV IgG is present in the sample. Only the control zone 309 turns into purple colour in case of HCV IgG free sample.

In compartment II (IgA), the sample zone 308' and the control zone 309' turn into purple colour in case that HCV IgA is present in the sample. Only the control zone 309' turns into purple colour in case of HCV IgA free sample.

### EXAMPLE 5: Oral H. pylori Antibody Detection Cup

The two compartment configuration (see Fig. 3 and Example 2 above) is used. The capacity of each of the compartments I and part II is at least 7.5 ml of sample. Gold conjugate A is mouse anti-human IgG colloidal gold conjugate immobilized on the conjugate releasing pad 303 of compartment I of the sample collection container 310, and gold conjugate B is mouse anti-human IgA colloidal gold conjugate immobilized on the conjugate releasing pad 303' of compartment II of the sample collection container 310. Two zones are located on each the detection strips 301, 301' of the two compartments; a sample zone 308 and a control zone 309. The sample zone 309, 309' is comprised of a mixture of recombinant and synthetic *H. pylori* specific antigens. The control zone 309, 309' of the two test strips 301, 301' is anti-mouse IgG.

In compartment I (IgG), the sample zone 308 and the control zone 309 turn into purple colour in case of *H. pylori* IgG availability in the sample. Only the control zone 309 turns into purple colour in case of *H. pylori* IgG free sample.

In compartment II (IgA), the sample zone 308' and the control zone 309' turn into purple colour in case of *H. pylori* IgA is present in the sample. Only the control zone 309'turns into purple colour in case of *H. pylori* IgA free sample.

### EXAMPLE 6: Oral HIV/HCV Antibody Detection Cup

The two compartment configuration (see Fig. 3 and Example 2) is used. The capacity of each of the compartments I and II is at least 7.5 ml of sample. Gold conjugate A is mouse anti-human IgG colloidal gold conjugate immobilized on the conjugate releasing pad 303 of compartment I of the sample collection container 310, and gold conjugate B is mouse anti-human IgA colloidal gold conjugate immobilized on the conjugate releasing pad 303' of compartment 11 of the sample collection container 310. Three zones are located at each the of detection strips of 301, 301' of the two compartments; a first sample zone 308, 308' a second sample zone 308, 308' and a control zone 309, 309'. The first sample zone 308, 308' comprised of a mixture of recombinant and synthetic HIV specific antigens, while the second sample zone 308, 308' is comprised of a mixture of recombinant and synthetic HCV specific antigens. The control zone 309, 309' of the two test strips 301, 301'is anti-mouse IgG.

In compartment 1 (IgG), the first sample zone 308 and the control zone 309 turn into purple colour in case that HIV IgG is present in the sample. The second sample zone 308 and the control zone 309 turn into purple colour in case that HCV IgG is present in the sample. Only the control zone 309 turns into purple colour in case of HIV/HCV IgG free sample.

In compartment II (IgA), the first sample zone 308' and the control zone 309' turn into purple colour in case that HIV IgA is present in the sample. The second sample zone 308 and the control zone 309 turn into purple colour in case of HCV IgA is present in the sample. Only the control zone 309' turns into purple colour in case of HIV/HCV IgA free sample.

### EXAMPLE 7: Oral HIV/HCV/H. pylori Antibody Detection Cup

The two compartment configuration (see Fig. 3and Example 2) is used. The capacity of each of the compartments I and II is at least 7.5 ml of sample. Gold conjugate A is mouse anti-human IgG colloidal gold conjugate immobilized on conjugate releasing pad 303 of compartment I of the sample collection container 310, and gold conjugate B is mouse anti-human IgA colloidal gold conjugate immobilized on the conjugate releasing pad 303' of compartment II of the sample collection container 310. Four zones are immobilized onto each the detection strips 301, 301' of the two parts; a first sample zone 308, 308', a second sample zone 308, 308', a third sample zone 308, 308' and a control zone 309, 309'. The first sample zone 308, 308' is comprised of a mixture of recombinant and synthetic HIV specific antigens, the second sample zone 308, 308' is comprised of a mixture of recombinant and synthetic HCV specific antigens, and the third sample zone 308, 308' is comprised of a mixture of recombinant and synthetic *H. pylori* specific antigens. The control zone 309, 309' of the two test strips 301, 301' is anti-mouse IgG.

In compartment I (IgG), the first sample zone 308 and the control zone 309 turn into purple colour in case that HIV IgG is present in the sample. The second sample zone 308 and the control zone turn into purple colour in case that HCV IgG is present in the sample. The third sample zone 308 and the control zone 309 turn into purple colour in case that *H. pylori* IgG is present in the sample. The first and the third sample zones 308 and the control zone 309 turn into purple colour in case that HIV and *H. pylori* IgG are present in the sample. The second and the third sample zones 308 and the control zone 309 turn into purple colour in case that HCV and *H. pylori* IgG are present in the sample. Only the control zone 309 turns into purple colour in case of HIV/HCV/*H. pylori* IgG free sample.

In compartment II (IgA), the first sample zone 308' and the control zone 309' turn into purple colour in case that HIV IgA is present in the sample. The second sample zone 308' and the control zone 309' turn into purple colour in case that HCV IgA is present in the sample. The third sample zone 308' and the control zone 309 turn into purple colour in case that *H. pylori* IgA is present in the sample. The first and third sample zone 308' and the control zone 309' turn into purple colour in case that HIV and *H. pylori* IgA are present in the sample. The second and third sample zones 308' and the control zone 309' turn into purple colour in case that HCV and *H. pylori* IgA are present in the sample. Only the control zone 309' turns into purple colour in case of HIV/HCV/*H. pylori* IgA free sample.

### References

(1) World Health Organization, HIV assays: operational characteristics (Phase I). Report 13: urine specimens, oral fluid (saliva) specimens. [Material originally distributed as WHO/BCT/02.08]
(2) J Chandler, N Robinson, and K Whiting, "Handling False Signals in Gold-Based Rapid Tests", IVD Technology 7, no. 2 (2001): 34-45; http://www.devicelink.com/ivdt/archive/01/03/002.html.
(3) TC Tisone et al., "Image Analysis for Rapid-Flow Diagnostics", IVD Technology 5, no. 5 (1999): 52-58; http://www.devicelink.com/ivdt/archive/99/09/010.html.
(4) J Chandler, T Gurmin, and N Robinson, "The Place of Gold in Rapid Tests", IVD Technology 6, no. 2 (2000): 37-49; http://www.devicelink.com/ivdt/archive/00/03/004.html

## Claims

1. An oral fluid immunochromatographic detection cup comprising:
(a) a sample-collecting container;
(b) at least one test device inside the container, which test device comprises at least one capture antigen recognized by an antibody to be detected and/or at least one control antibody;
(c) at least one conjugate releasing pad separated from the test device, which conjugate releasing pad comprises at least one anti-antibody colloidal gold conjugate;
(d) at least two sample absorbent pads linked to the test device at different positions.

2. The detection cup according to claim 1 comprising:
(a) a sample-collecting container comprising at least two compartments;
(b) at least one test device inside each compartment, which test device comprises at least one capture antigen recognized by an antibody to be detected and/or at least one control antibody;
(c) at least one conjugate releasing pad inside each compartment separated from the test device, which conjugate releasing pad comprises at least one anti-antibody colloidal gold conjugate;
(d) at least two sample absorbent pads linked to each test device at different positions.

3. The detection cup according to claim 1 or 2, wherein the test device further comprises a nitrocellulose membrane on which the capture antigen and/or the control antibody is immobilized.

4. The detection cup according to any of the preceding claims, wherein the antibody to be detected is a human antibody selected from HIV-1 IgG, HIV-1 IgA, HIV-2 IgG, HIV-2 IgA, HCV IgG, HCV IgA, *H. pylori* IgG, and *H. pylori* IgA.

5. The detection cup according to any of the preceding claims, wherein the control antibody is anti-mouse IgG.

6. The detection cup according to any of the preceding claims, wherein the anti-antibody colloidal gold conjugate is mouse anti-human IgG or mouse anti-human IgA or specific antigen colloidal gold conjugate.

7. The detection cup according to any of the preceding claims, wherein the conjugate releasing pad is located at the internal surface of the container wall.

8. The detection cup according to any of the preceding claims, wherein a first sample absorbent pad is located at the bottom and a second sample pad is located at the top of the container.

9. The detection cup according to claim 8, wherein the sample absorbent pad at the top is fitted into a container cap.

10. The detection cup according to claim 8 or 9, wherein the sample absorbent pad at the top has a capacity of absorbing at least 15 ml of sample.

11. A use of the oral fluid immunochromatographic detection cup according to any of claims 1 to 10 for detecting a human antibody selected from HIV-1 IgG, HIV-1 IgA, HIV-2 IgG, HIV-2 IgA, HCV IgG, HCV IgA, *H. pylori* IgG, and *H. pylori* IgA in oral fluid of a subject, preferably human.

12. The use according to claim 11 for simultaneously detecting HIV-1 IgG, HIV-1 IgA, HIV-2 IgG, HIV-2 IgA, HCV IgG, and HCV IgA.

13. The use according to claims 11 to 12 for simultaneously detecting of HIV-1 IgG, HIV-1 IgA, HIV-2 IgG, HIV-2 IgA, HCV IgG, *H. pylori* IgG, and *H. pylori* IgA

14. The use according to any of claims 11 to 13 for the diagnosis and monitoring of a subject infected with HIV-1, HIV-2, HCV and/or *H. pylori.*
